Europäisches Patentamt

European Patent Office

Office européen des brevets

(11) Publication number: **0 231 974**
**A1**

## (12) EUROPEAN PATENT APPLICATION

(21) Application number: **87200131.8**

(22) Date of filing: **29.01.87**

(51) Int. Cl.⁴: **A41B 13/02**

(30) Priority: **03.02.86 NL 8600259**

(43) Date of publication of application:
**12.08.87 Bulletin 87/33**

(84) Designated Contracting States:
**AT BE CH DE ES FR GB GR IT LI LU NL SE**

(71) Applicant: **Ancilla B.V.**
**Hoogveld 25 P.O. Box 19**
**NL-6590 AA Gennep(NL)**

(72) Inventor: **Hugen, G.H.M., Ir.**
**Bovensteweg 6**
**NL-6585 KC Mook(NL)**

(74) Representative: **Noz, Franciscus Xaverius, Ir.**
**et al**
**Boschdijk 155 P.O. Box 645**
**NL-5600 AP Eindhoven(NL)**

(54) **Disposable napkin.**

(57) Disposable napkin provided with a back skin at least substantially impermeable to liquid, a liquid-permeable upper skin and a layer of liquid-absorbing material, said layer being enclosed between the back skin and the upper skin, said skins being attached together around the layer of absorbing material, whereby the layer of absorbing material, seen in top view, is at least substantially shaped like a pair of isosceles trapeziums joining each other with the shorter sides of the parallel sides, whilst the longer of the two parallel sides of the one trapezium is approximately twice as long as the longer side of the two parallel sides of the other trapezium and the total height of the two trapeziums lies between 20 and 25 cm in unstretched condition.

EP 0 231 974 A1

## Disposable napkin.

The invention relates to a disposable napkin provided with a back skin at least substantially impermeable to liquid, a liquid-permeable upper skin and a layer of liquid-absorbing material, said layer being enclosed between the back skin and the upper skin, said skins being attached together around the layer of absorbing material. Such disposable napkins are generally relatively voluminous, whilst they also have a bad fit. In many cases a disposable napkin having substantially less liquid-absorbing material will be sufficient.

According to the invention the layer of absorbing material, seen in top view, is at least substantially shaped like a pair of isosceles trapeziums joining each other with the shorter sides of the parallel sides, whilst the longer of the two parallel sides of the one trapezium is approximately twice as long as the longer side of the two parallel sides of the other trapezium, whilst the total height of the two trapeziums lies between ± 20 and ± 25 cm in unstretched condition.

Such a napkin has a good fit, is not unpleasant to wear and in practice it has appeared that in many cases such a napkin contains sufficient liquid-absorbing material to meet the need.

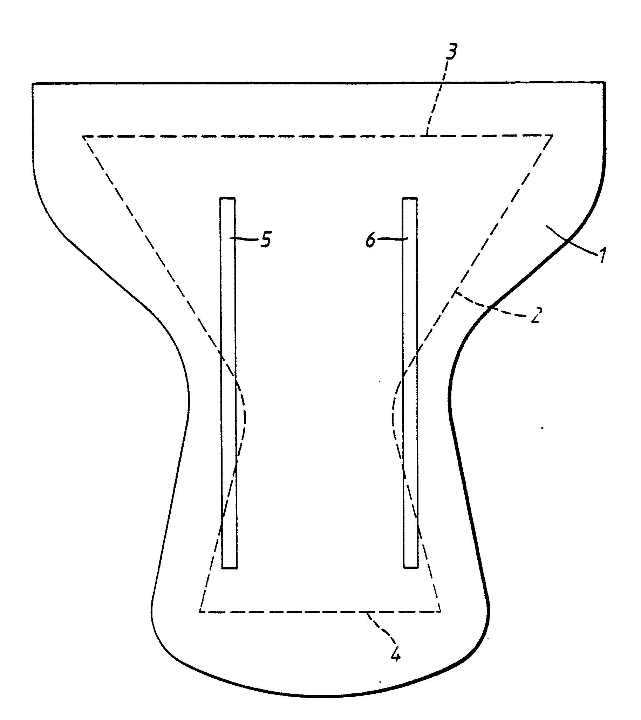

The invention will be more fully explained hereinafter with reference to the accompanying figure, in which a napkin according to the invention is illustrated in top view.

The figure illustrates a top view of a napkin which is built up in the usual manner from a back skin 1 at least substantially impermeable to liquid and a liquid-permeable upper skin located thereunder, seen in the figure, whilst between said two skins there is located a layer 2 of absorbing material. The boundary of said layer 2 of absorbing material is indicated by a dotted line in the figure. As appears from the figure said layer 2 of absorbing material is at least substantially shaped like a pair of isosceles trapeziums, whereby the shorter sides of said trapeziums, which have the same length, join each other. The longer side 3 of the two sides of the one trapezium extending parallel to each other, is about twice as long as the longer side 4 of the two sides of the other trapezium extending parallel to each other. The length of the longer side 3 preferably lies between 20 and 25 cm, and in practice ± 23 cm has appeared to be a good length. The length of the shorter side 4 is ± 11½ cm then.

The total height of the two trapeziums, i.e. the distance between the two sides 3 and 4 extending parallel to each other lies between 20 and 25 cm and is preferably ± 23 cm.

At the narrowest part the width of the layer of absorbing material is ± 6 cm.

The back skin 1 and the correspondingly shaped upper skin of liquid-permeable material located under the back skin protrude ± 2-3 cm from around the layer of absorbing material and said edges of the two skins protruding from the layer of absorbing material are attached together in the usual manner.

Furthermore a pair of strips 5 and 6, consisting of elastic material, are attached to the side of the back skin 1 facing away from the layer of absorbing material. Thereby the strips are attached to the skin in the stretched position illustrated in the figure, so that when the napkin is released from the stretched position illustrated in the figure the strips 5 and 6 contract. The strips 5 and 6 consisting of elastic material and extending perpendicular to the sides 3 and 4 are thereby provided in such a manner that at the narrowest part of the layer of absorbing material they are located at a distance of only 10-15 mm from the layer of absorbing material.

When the napkin is put on care must be taken that the part with the long side 3 is at the front of the body. The elastic strips 5 and 6 provide a good fit of the napkin in the crotch.

In practice it has appeared that such a napkin which takes up little space is not unpleasant to wear for the user and provides sufficient protection in many cases.

## Claims

1. Disposable napkin provided with a back skin at least substantially impermeable to liquid, a liquid-permeable upper skin and a layer of liquid-absorbing material, said layer being enclosed between the back skin and the upper skin, said skins being attached together around the layer of absorbing material, characterised in that the layer of absorbing material, seen in top view, is at least substantially shaped like a pair of isosceles trapeziums joining each other with the shorter sides of the parallel sides, whilst the longer of the two parallel sides of the one trapezium is approximately twice as long as the longer side of the two parallel sides of the other trapezium and the total height of the two trapeziums lies between 20 and 25 cm in unstretched condition.

2. Disposable napkin according to claim 1, characterised in that in unstretched condition the total height of the two trapeziums is ± 23 cm.

3. Disposable napkin according to claim 1 or 2, characterised in that the length of the longest side of the sides of the two trapeziums extending parallel to each other lies between 21 and 25 cm and is preferably 23 cm.

4. Disposable napkin according to any of the preceding claims, characterised in that the length of the two shorter sides of the sides of the trapeziums extending parallel to each other is 6 to 7 cm.

5. Disposable napkin according to any of the preceding claims, characterised in that strips consisting of elastic material are attached, in stretched condition, to the back skin, such that said strips extend perpendicularly to the sides of the two trapeziums extending parallel to each other, whilst at the narrowest part of the layer of absorbing material the distance between the outer boundary walls of the layer of absorbing material and the strip consisting of elastic material is equal to or smaller than 15 mm.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 337 771 (PIENIAK et al.)<br>* Figure 11; column 6, lines 35-52 * | 1,5 | A 41 B 13/02 |
| A | US-A-3 860 003 (BUELL)<br>* Figure 1; column 5, lines 42-55 * | 1,3-5 | |
| A | FR-A-2 421 571 (JOHNSON & JOHNSON)<br>* Figures 1-2; pages 7,8 * | 1 | |
| A | EP-A-0 059 015 (THE PROCTER & GAMBLE CO.) | | |
| A | GB-A-2 080 093 (MÖLNLYCKE AB) | | **TECHNICAL FIELDS SEARCHED (Int. Cl.4)** |
| A | US-A-3 809 089 (HEDSTROM et al.) | | A 41 B<br>A 61 F |

The present search report has been drawn up for all claims

| Place of search | Date of completion of the search | Examiner |
|---|---|---|
| THE HAGUE | 16-04-1987 | RIEGEL R.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82